# EUROPÄISCHE PATENTSCHRIFT

(11) **EP 0 576 112 B1**
(45) Veröffentlichungstag und Bekanntmachung des Hinweises auf die Patenterteilung: **21.05.1997**
(21) Anmeldenummer: 93250181.0
(22) Anmeldetag: 21.06.1993
(51) Int. Cl.: A61K 6/087, C08G 73/04, A61K 6/083

(54) **Dentalmaterial**
Dental material
Matériau dentaire

(30) Priorität: 25.06.1992 DE 4220958
(43) Veröffentlichungstag der Anmeldung: 29.12.1993
(73) Patentinhaber: IVOCLAR AG, FL-9494 Schaan (LI)
(72) Erfinder: Rheinberger, Volker, Dr., FL-9490 Vaduz (LI); Salz, Ulrich, Dr., D-8995 Weissenberg (DE); Burtscher, Peter, Dr., A-6714 Nütziders (AT)
(74) Vertreter: UEXKÜLL & STOLBERG

(56) Entgegenhaltungen:
- EP-A- 0 195 224
- DE-A- 1 544 837
- US-A- 3 538 024
- US-A- 4 093 555

## Beschreibung

Die Erfindung betrifft ein Dentalmaterial, welches in einer ersten Stufe zu einer elastischen Phase, in der das Material mechanisch bearbeitet oder Überschüsse entfernt werden können, und in einer zweiten Stufe zu seiner endgültigen Form aushärtbar ist.

Dentalmaterialien, die über einen praxisrelevanten Verarbeitungszeitraum, der nicht zu lang und nicht zu kurz sein darf, eine elastische Phase aufweisen, sind für spezielle Anwendungsbereiche besonders gut geeignet und sind deshalb den derzeit auf dem Markt befindlichen Materialien vorzuziehen.

Diese speziellen Anwendungsbereiche sind zum einen der Bereich der temporären Kronen und Brücken zur provisorischen Versorgung von prothetischen Arbeiten sowie der temporären Füllungen zur provisorischen Versorgung vor allem von mehreren, nebeneinander liegenden Füllungen. Zum anderen sind es Kunststoff-Dentalzemente zum Einzementieren eines zahnärztlichen Werkstücks, deren Überschüsse in dieser elastischen Phase einfach entfernt werden können.

Die für temporäre Kronen und Brücken benutzten Materialien, die zur Zeit auf dem Markt sind, sind vorwiegend selbsthärtende Polymethacrylat/Methacrylatsysteme, Composite auf Methacrylatbasis oder andere Polymersysteme wie zum Beispiel Epiminsysteme.

Zur Anfertigung einer temporären oder provisorischen Krone oder Brücke wird das angemischte temporäre Kronen- und Brückenmaterial in einen entsprechenden Abdruck gegeben, mit dem das Material in der vorgegebenen Form einer Krone oder Brücke auf den oder die zu versorgenden Zahnstümpfe gebracht wird. In diesem Abdruck härtet das Material dann aus. Zu einem bestimmten Zeitpunkt jedoch, an welchem das Material noch nicht vollständig ausgehärtet ist, muß der Abdruck, und zwar zusammen mit der temporären Versorgung, entfernt werden. Bei den bis jetzt verwendeten Materialien ist der hierfür verfügbare Zeitraum aber sehr klein und wird oft verpaßt. Die Gründe hierfür sind, daß die Aushärtung dieser Materialien im Abdruck nicht kontrollierbar ist und daß sie außerdem von vielen Störfaktoren wie Anmischverhältnisse, Temperatur, Feuchtigkeit usw. beeinflußt wird.

Das hat zur Folge, daß, wenn man die Krone zu früh entfernt, das Material entweder noch zähflüssig oder nicht formstabil ist, d.h., die temporäre Versorgung, also die provisorische Brücke oder Krone muß noch einmal angefertigt werden. Wartet man andererseits zu lange, so ist die temporäre Krone bzw. Brücke nicht mehr vom Stumpf entfernbar, vor allem dann nicht, wenn untersichgehende Stellen vorhanden sind. Dazu kommt, daß in der Regel mit Verwendung dieser Materialien auch Temperaturprobleme verbunden sind. Die Polymerisationsreaktion, die wie erwähnt nicht kontrollierbar ist, verläuft exotherm, wobei soviel thermische Energie frei wird, daß es zur Schädigung der Pulpa kommen kann.

Ähnlich liegen die Probleme bei Verwendung als temporäre Füllungsmaterialien. Bei großen, nebeneinander liegenden Kavitäten (vorwiegend MOD), die mit laborgefertigten Inlays versorgt werden, nimmt man die provisorische Versorgung der Kavitäten häufig so vor, daß man die nebeneinander liegenden Kavitäten en bloc versorgt. Dazu wird das selbsthärtende temporäre Material in die isolierten Kavitäten eingebracht und wiederum zu einem Zeitpunkt aus diesen Kavitäten entfernt, bei welchem das Material noch eine gewisse Elastizität aufweist. Wird dieser Zeitpunkt nicht genau abgepaßt, so treten Probleme auf, wie sie bereits für die Anwendung als temporäres Kronen- und Brückenmaterial beschrieben wurden. Meistens ist die Aushärtung zu weit fortgeschritten, so daß die temporäre Füllung nicht mehr entfernt werden kann. Außerdem sind auch hier Probleme bezüglich der Temperaturbelastung zu erwarten.

Nachdem die temporäre Füllung aus der Kavität entfernt worden ist, werden untersichgehende Stellen entfernt und dann wird das Werkstück mit einem temporären Zement eingegliedert.

Eine ähnliche Technik hat sich in jüngster Zeit für die Herstellung von chairside (also am Zahnarztstuhl) oder labside (im Dentallabor) gefrästen Keramik-Inlays entwickelt. Wie oben beschrieben, wird die isolierte Kavität mit einem sogenannten Proinlay-Material gefüllt und das Material so modelliert, wie die letztendliche Inlay-Füllung geformt sein soll. Meistens hat man auch hier Probleme, das Proinlay aus der Kavität zu entfernen, einerseits wegen der untersichgehenden Stellen, andererseits wegen der geforderten hohen Paßgenauigkeit. Danach wird das Proinlay mit einer Kopierfräse in das definitive Keramikinlay transferiert. Chairside wird das Keramikinlay sofort eingegliedert, bei der Labside-Technik wird die Kavität durch ein oben beschriebenes Material versorgt.

Aus den dargelegten Problemem wird deutlich, daß sowohl für temporäre Kronen- und Brückenmaterialien als auch für temporäre Füllungsmaterialien Bedarf nach einem Material besteht, das in einer ersten Stufe kontrolliert bis zu einer bestimmten Elastizität aushärtet und das in dieser Phase über einen prasixrelevanten Zeitraum stabil bleibt und damit leicht vom Stumpf bzw. aus der Kavität entfernt werden kann. Es kann dann in dieser Form bereits bearbeitet und finiert und anschließend in einer zweiten Stufe zu seiner Endform ausgehärtet werden.

Der andere oben erwähnte Anwendungsbereich ist ein Composite-Zement mit möglicher Überschußentfernung: Bei der Einzementierung von zahnärztlichen Werkstücken wie z.B. Keramik-Inlays, Keramik- oder Metallkronen mit zahnfarbenen Composite-Zementen ist es sehr schwierig, Zementüberschüsse zu erkennen und möglichst schonend zu entfernen.

Werden die Überschüsse in unausgehärtetem Zustand entfernt, wird meist der Zement aus der Zementfuge mit herausgenommen, was zur Folge hat, daß Unterschüsse erzeugt werden. Wenn man andererseits den Zementüberschuß in ausgehärtetem Zustand zu entfernen versucht, werden meist der Zement sowie auch die Zahnhartsubstanz sehr stark in Mitleidenschaft genommen.

Es ist deutlich, daß auch für diesen Anwendungsbereich Bedarf nach einem Material besteht, das in einer ersten Stufe kontrolliert zu einer elastischen Phase aushärtet, in der das Werkstück zwar bereits in der richtigen Position fixiert wird, sich aber die Überschüsse des Zements mit einem scharfen Instrument wie einem Skalpell wegschneiden lassen.

Aus EP-B-195 224 der Anmelderin ist ein Dentalwerkstoff zur Herstellung von künstlichen Zähnen oder Zahnteilen wie Kronen oder Inlays aus zwei getrennten, bei Gebrauch miteinander zu vermischenden Komponenten bekannt, bei dem in einer ersten Stufe ein polyfunktionelles Isocyanat mit einem Polyalkohol unter Zuhilfenahme von Zinnkatalysatoren zu einem Polyurethan kondensiert, das als elastische Phase vorliegt, die über einen längeren Zeitraum stabil bleibt.

In einer zweiten Stufe wird dann mindestens eine im Gesamtsystem enthaltene Methacrylatverbindung durch Heiß-, Kalt- oder Lichtpolymerisation zu künstlichen Zähnen oder Zahnteilen ausgehärtet.

Ferner sind aus EP-A-410 199 (Bayer) mehrstufig aushärtbare Kunststoffe bekannt, die aus mindestens einem Silikonpolyether, mindestens einem radikalisch härtenden Monomeren sowie mindestens einem Katalysator für die Heiß-, Kalt- oder Lichtpolymerisation und mindestens einem Katalysator zur Kondensation des Silikopolyethers bestehen. Derartige Kunststoffe auf Silikopolyetherbasis haben jedoch den Nachteil, daß die Silikopolyether die Polymerisation der radikalisch härtenden Monomere negativ beeinflussen. Die Polymerisation der zweiten Stufe, d.h. der Monomeren bzw. der Methacrylate ist unvollständig, was sich wiederum negativ auf die physikalischen Eigenschaften der ausgehärteten Kunststoffe auswirkt. Insbesondere sind die Biegemodule der ausgehärteten Kunststoffe unbefriedigend.

Bekannt sind auch - wie erwähnt - Epimine sowie ihr Einsatz im Dentalbereich. Sie werden insbesondere als Abformmaterialien sowie als temporäre Kronen- und Brückenmaterialien verwendet. In Abformmaterialien werden Epimine mit einem Molekulargewicht von ca. 6000 verwendet, wie in DE-B-15 44 837 beschrieben. Sie polymerisieren zu einer elastischen Phase und erfüllen dadurch ihren Zweck. In einem temporären Kronen- und Brückenmaterial (US-A-3 453 242 und US-A-4 093 555) werden kürzerkettige Epimine mit einem Molekulargewicht von ca. 500 eingesetzt. Diese polymerisieren zu einer harten Masse, wobei während der Polymerisation in einem kurzen Zeitraum eine elastische Phase durchlaufen wird.

Es sind keine Veröffentlichungen bekannt, in denen mit Epiminen allein oder in Kombination mit anderen Polymerisationssystemen ein Produkt erhalten wird, welches über einen längeren Zeitraum in einer elastischen Phase verbleibt und anschließend nach entsprechender Aktivierung in einen harten Zustand übergeht.

Aufgabe der Erfindung ist es, ein Dentalmaterial verfügbar zu machen, welches in einer ersten Stufe zu einer elastischen Phase, in der das Material mechanisch bearbeitet oder Überschüsse entfernt werden können, und in einer zweiten Stufe zu seiner endgültigen Form aushärtbar ist. Aufgabe ist ferner ein Dentalmaterial zu schaffen, das sowohl die Herstellung von künstlichen, provisorischen Zahnteilen als auch die Befestigung bzw. Einzementierung von künstlichen Zähnen und Zahnteilen aus Keramik oder Metall in zwei Stufen zuläßt, wobei die erste Stufe genügend lange eine solche Elastizität gewährleistet, daß die provisorischen Zahnteile ausgearbeitet und die bei der Einzementierung fertiger Zähne oder Zahnteile anfallenden Überschüsse problemlos entfernt werden können. Aufgabe ist insbesondere ein Dentalmaterial verfügbar zu machen, welches sich als Zahnersatzmaterial für temporäre Kronen und Brücken, als temporäres Füllungsmaterial sowie als Proinlay-Material für die CAM-Frästechnik ebenso wie als Composite-Zement zur Einzementierung eines zahnärztlichen Werkstücks eignet.

Es war überraschend, daß man ein derartiges Material durch Kombination eines Epimin- mit einem Methacrylat-Polymersystem erhält.

Zur Lösung der Aufgabe wird daher ein Dentalmaterial vorgeschlagen, welches in einer ersten Stufe zu einer elastischen Phase, in der das Material mechanisch bearbeitet oder Überschüsse entfernt werden können, und in einer zweiten Stufe zu seiner endgültigen Form aushärtbar ist und welches gemäß Hauptanspruch
(a) mindestens ein polyfunktionelles Epimin (Aziridin),
(b) mindestens ein ethylenisch ungesättigtes Monomer,
(c) mindestens einen Katalysator für die Heiß-, Kalt- oder Lichtpolymerisation des ethylenisch ungesättigten Monomeren und
(d) mindestens einen Katalysator zur Beschleunigung der Polymerisation des Epimins (a), welcher jedoch die Polymerisation von (b) nicht beeinflußt,
enthält.

Das Material liegt bevorzugt in zwei getrennten Komponenten, einer ersten Komponente oder Basenkomponente und einer zweiten Komponente oder Basenaktivatorkomponente vor, die bei Gebrauch miteinander vermischt werden, vorzugsweise in einem Verhältnis 1:1.

Auch ist es bevorzugt, daß das Material einen anorganischen und/oder organischen Füllstoff enthält.

Im Unterschied zu den oben genannten bekannten interstitiellen bzw. Zwischenraumpolymerisaten, bei denen die erste Stufe, d.i. die Stufe der elastischen Phase durch Polykondensation oder Kondensationspolymerisation eines polyfunktionellen Isocyanats mit einem Polyalkohol erhalten wird, wird erfindungsgemäß die elastische Phase nach einem völlig anderen Polymerisationsmechanismus erreicht, und zwar durch kationische Polymerisation. Bei dieser kationischen Polymerisation werden polyfunktionelle Imine (synonym für Aziridin - bzw. Ethyleniminverbindungen) zu einer elastischen Phase polymerisiert.

Die in dem Dentalmaterial der Erfindung für die Polymerisation zur elastischen Phase verwendeten Epimine werden nach dem in DE-PS-1 544 837 beschriebenen Verfahren hergestellt.

Als Vernetzer bzw. Katalysator für die kationische Polymerisation werden übliche kationisierende Aktivatoren oder Starter, vorzugsweise Arylsulfonsäureester gemäß der DE-PS-1 544 837, insbesondere 2,5-Dichlorbenzolsulfonsäuremethylester sowie spezielle Sulfoniumsalze gemäß US-A-4 167 618, entsprechend DE-A- 25 15 593, verwendet.

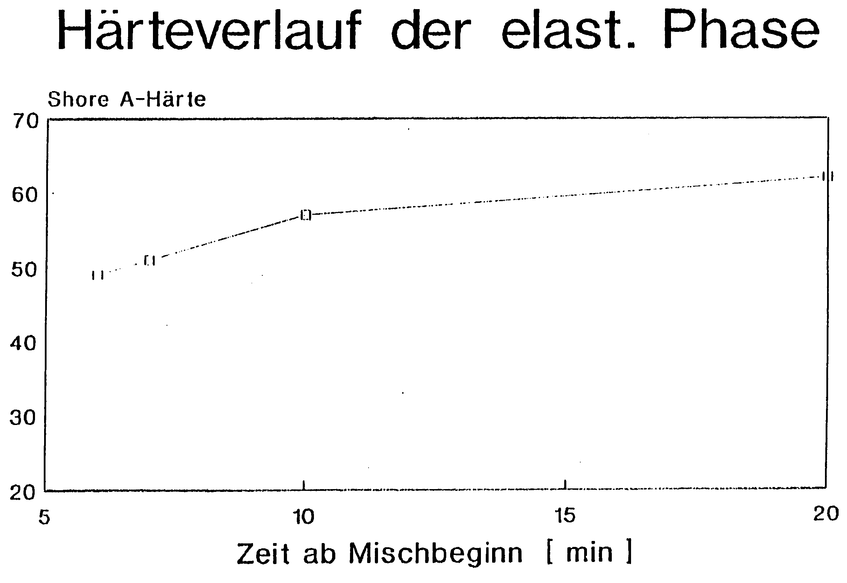

Die bei dieser kationischen Polymerisation erzielte elastische Phase bleibt über einen praxisrelevanten Zeitraum von etwa 3 bis 30 Minuten stabil. Dies ist in Figur 1 durch den Härteverlauf der elastischen Phase in Abhängigkeit von der Zeit ab Mischbeginn dargestellt. Die Polymerisation der ersten Stufe bis zur Erreichung der elastischen Phase erfolgt bei einer Temperatur von 20 bis 37°C.

Das Dentalmaterial der Erfindung muß ferner mindestens ein ethylenisch ungesättigtes Monomer enthalten. Geeignete derartige ethylenisch ungesättigte Monomere sind Methacrylate und Acrylate, vorzugsweise mono- oder polyfunktionelle Methacrylate, insbesondere Isobutylmethacrylat, Tetraethylenglycoldimethacrylat, Triethylenglycoldimethacrylat, Diethylenglycoldimethacrylat, Ethylenglykoldimethacrylat, Polyethylenglykoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4(3-methacryloxy-2-hydroxy)-phenylpropan(Bis-GMA) sowie Reaktionsprodukte aus Di- und/oder Triisocyanaten und OH-gruppenhaltigen Methacrylaten.

Als Katalysator für die Heißpolymerisation des ethylenisch ungesättigten Monomeren kommen die üblichen in Frage, beispielsweise Peroxide wie Dibenzoylperoxid, Dilauroylperoxid, tert.-Butylperoctoat, tert.-Butylperbenzoat, aber auch α,α'-Azo-bis-(isobutyroethylester), AIBN, Benzpinakol und 2,2'-Dimethylbenzpinakol.

Als Katalysator für die Kaltpolymerisation des ethylenisch ungesättigten Monomeren kommen die üblichen in Frage, insbesondere Amin/Peroxid-Systeme (z.B. Dibenzoylperoxid/N,N-Dimethyl-p-toluidin).

Als Katalysator für die Lichtpolymerisation des ethylenisch ungesättigten Polymeren eignen sich Benzophenon und seine Derivate, Benzoin und seine Derivate, Acylphosphinoxide, insbesondere α-Diketone wie Campherchinon, gegebenenfalls in Verbindung mit einem Amin als Reduktionsmittel.

Das Dentalmaterial enthält vorzugsweise mindestens einen anorganischen und/oder organischen Füllstoff. Hierfür eignen sich beispielsweise AEROSIL® (pyrogenes SiO₂), Bariumsilikatglas, das vorzugsweise silanisiert ist, gefällte Kieselsäure, durch Sol-Gel-Prozeß erhaltene Oxide bzw. Mischoxide, röntgenopake Lanthanidenverbindungen wie z.B. YbF₃, Strontium-, Barium- und Li-Al-Silikat-Gläser, wobei die oxidischen Verbindungen vorzugsweise silanisiert werden. Ferner können noch Stabilisatoren, Farbstoffe oder andere Hilfsstoffe enthalten sein.

Ebenfalls eignen sich Präpolymere, die aus einem oder mehreren der oben genannten Füllstoffe sowie einem oder mehreren ethylenisch ungesättigten Monomeren durch Heißhärtung und anschließende Mahlung erhalten werden.

Die Herstellung des Dentalmaterials der Erfindung erfolgt durch einfaches Vermischen der Bestandteile, bevorzugt im Komponenten-Verhältnis 1:1, wobei das Aziridin (a) nur in der ersten Komponente, der kationisierende Starter (d) nur in der zweiten Komponente eingesetzt wird. Das ethylenisch ungesättigte Monomer befindet sich vorzugsweise sowohl in der ersten als auch in der zweiten Komponente. Der die Polymerisation des ethylenisch ungesättigten Polymeren aktivierende Katalysator (c) wird bei der Licht- und Heißpolymerisation vorzugsweise in der ersten Komponente eingesetzt, das für die Kaltpolymerisation verwendete Amin/Peroxid-Katalysatorsystem (c) wird getrennt in den beiden Komponenten eingesetzt.

Das polyfunktionelle Epimin (a) wird, bezogen auf die erste Komponente, die Basenkomponente, vorzugsweise in einer Menge von 5 bis 70 Gew.-%, besonders bevorzugt 6 bis 40 Gew.-% eingesetzt. Bezogen auf das gesamte Gemisch, also die angemischten Komponenten, wird (a) vorzugsweise in einer Menge von 2,0 bis 35 Gew.-%, besonders bevorzugt 2,5 bis 20 Gew.-% eingesetzt.

Das ethylenisch ungesättigte Monomer (b) wird vorzugsweise in beiden Gemischen eingesetzt, und zwar in einem Bereich von 10 bis 80 Gew.-%, vorzugsweise 15 bis 50 Gew.-% der gesamten Mischung.

Die Katalysatoren (c) werden vorzugsweise in einer Menge von 0,01 bis 5 Gew.-%, bezogen auf das gesamte Material, eingesetzt.

Der kationisierende Starter (d) wird vorzugsweise in einer Menge von 0,1 bis 5 Gew.-%, bezogen auf die zweite Komponente eingesetzt, bezogen auf das gesamte Material in einer Menge von 0,05 bis 2,5 Gew.%.

Der Füllstoff wird vorzugsweise in Mengen von 20 bis 80 Gew.-%, bezogen auf das gesamte Material, eingesetzt.

In den folgenden Beispielen wurden zwei unterschiedliche Epimine, hergestellt gemäß DE-B-15 44 837, eingesetzt. In einem Fall wurde Polytetrahydrofuran mit einem mittleren Molekulargewicht von 1200 als Diolkomponente verwendet (nachfolgend als PTHF-Epimin bezeichnet), im anderen Fall wurde Polyethylenglykol mit einem mittleren Molekulargewicht von 6000 als Diolkomponente verwendet (nachfolgend als PE-Epimin bezeichnet).

### Beispiel 1

### Temporäres Kronen- und Brückenmaterial

### Basenaktivatorpaste:

In 8,8 Gew.-% Triethylenglykoldimethacrylat und 17,5 Gew.-% UDMA* wurden 1,5 Gew.-% 2,5-Dichlorbenzolsulfonsäuremethylester und 0,1 Gew.-% 3,5-Di-t-butyl-4-hydroxytoluol gelöst. Danach wurden 66,0 Gew.-% Ba-Silikatglas (silanisiert) und 6,1 Gew.-% AEROSIL® Ox-50 (silanisiert) in einem Kneter zugemischt.

### Basenpaste:

In 4,6 Gew.-% Triethylenglykoldimethacrylat und 9,16 Gew.-% UDMA* wurden 0,08 Gew.-% Campherchinon und 0,16 Gew.-% N-2-Cyanoethyl-N-methylanilin gelöst. Danach wurden 5,6 Gew.-% PTHF-Epimin, 8,4 Gew.-% PE-Epimin und 72,0 Gew.-% Ba-Silikatglas (silanisiert) in einem Kneter zugemischt.
* *Reaktionsprodukt aus 1 Mol 2,2,4-Trimethylhexamethylendiisocyanat mit 2 Mol Hydroxyethylmethacrylat (HEMA).*

Auf diese Weise wurde ein gut mischbares Zweikomponenten-Dentalmaterial erhalten. Nach dem Anmischen im Verhältnis 1:1 härtete das Material bei einer Temperatur von 37°C nach 2,1 Minuten zu einer elastischen Phase aus.

Die Messung der Härte in Abhängigkeit von der Zeit ergab:

| Zeit ab Mischbeginn [Min] | Shore A-Härte |
|---|---|
| 6 | 49 |
| 7 | 51 |
| 10 | 57 |
| 20 | 62 |

Diese Zahlen zeigen, daß die elastische Phase über einen Zeitraum von mindestens etwa 20 Minuten stabil ist (Figur 1).

Danach wurde das Material durch 40 Sekunden lange Bestrahlung mit einem Halogenlichtgerät (Heliomat®) in seine endgültige Form überführt. Das erhaltene Material hatte folgende Eigenschaften:

| | |
|---|---|
| Biegefestigkeit: | 59 MPa |
| Biegemodul: | 3200 MPa |

### Beispiel 2

### Befestigungscomposite mit möglicher Überschußentfernung

### Basenaktivatorpaste:

In 14,2 Gew.-% Triethylenglykoldimethacrylat und 14,14 Gew.-% ethoxyliertem Bis-glycidylmethacrylat (Bis-GMA) wurden 1,8 Gew.-% 2,5-Dichlorbenzolsulfonsäuremethylester, 0,8 Gew.-% Benzoylperoxid (50 %-ig) und 0,06 Gew.-% 3,5-Di-t-butylhydroxytoluol gelöst. Danach wurden 69,0 Gew.-% Ba-Silikatglas (silanisiert) in einem Kneter zugemischt.

### Basenpaste:

In 11,6 Gew.-% Triethylenglykolmethaycrylat und 11,6 Gew.-% ethoxyliertem Bis-GMA wurden 0,07 Gew.-% Campherchinon, 0,07 Gew.-% N-Cyanoethyl-N-methylanilin, 0,03 Gew.-% 3,5-Di-t-butyl-4-hydroxytoluol und 0,1 Gew.-% N,N-Diethanol-3,5-di-t-butylanilin gelöst. Danach wurden 3,5 Gew.-% PTHF-Epimin, 3,5 Gew.-% PE-Epimin und 69,53 Gew.-% Ba-Silikatglas (silanisiert) in einem Kneter zugemischt.

Auf diese Weise wurde ein gut mischbarer Zweikomponenten-Composite-Zement erhalten. Nach dem Anmischen im Verhältnis 1:1 härtete das Material bei einer Temperatur von 37°C nach 3 Minuten zu einer elastischen Phase aus. Nach weiteren 2 Minuten setzte die Selbsthärtung des Amin/Peroxidsystems ein.

## Patentansprüche

1. Dentalmaterial, welches in einer ersten Stufe zu einer elastischen Phase, in der das Material mechanisch bearbeitet oder Überschüsse entfernt werden können, und in einer zweiten Stufe zu seiner endgültigen Form aushärtbar ist, enthaltend
(a) mindestens ein polyfunktionelles Epimin (Aziridin),
(b) mindestens ein ethylenisch ungesättigtes Monomer,
(c) mindestens einen Katalysator für die Heiß-, Kalt- oder Lichtpolymerisation des ethylenisch ungesättigten Monomers und
(d) mindestens einen Katalysator zur Beschleunigung der Polymerisation des Epimins (a), welcher jedoch die Polymerisation von (b) nicht beeinflußt.

2. Material nach Anspruch 1, **dadurch gekennzeichnet**, daß es in zwei getrennten Komponenten vorliegt, nämlich in einer ersten oder Basenkomponente und in einer zweiten oder Basenaktivatorkomponente, die bei Gebrauch miteinander vermischt werden, vorzugsweise in einem Verhältnis 1:1.

3. Material nach Anspruch 2, **dadurch gekennzeichnet**, daß
(a) nur in der ersten Komponente,
(b) mindestens in der ersten und vorzugsweise auch in der zweiten Komponente,
(c) mindestens in der ersten Komponente und
(d) nur in der zweiten Komponente enthalten ist.

4. Material nach einem oder mehreren der vorhergehenden Ansprüche, **dadurch gekennzeichnet**, daß es außerdem mindestens einen anorganischen und/oder organischen Füllstoff enthält.

5. Material nach einem oder mehreren der vorhergehenden Ansprüche, **dadurch gekennzeichnet**, daß die elastische Phase in einem praxisrelevanten Zeitraum von mindestens etwa 3 bis 30 Minunten stabil bleibt.

6. Material nach einem oder mehreren der vorhergehenden Ansprüche, **dadurch gekennzeichnet**, daß das polyfunktionelle Epimin (a) eine Ethyleniminverbindung ist, bei der an den Enden oder in Seitenketten von im wesentlichen linearen Polyethern, Polythioethern oder gesättigten Polyestern mit einem durchschnittlichen Molgewicht von 1000 bis 25 000 durchschnittlich mehr als ein Ethyleniminrest der allgemeinen Formel 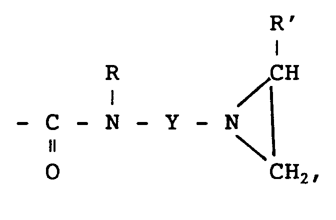 in der R und R' ein Wasserstoffatom oder einen Alkylrest und Y einen zweiwertigen organischen Rest bedeuten, eingeführt worden ist.

7. Material nach einem oder mehreren der vorhergehenden Ansprüche, **dadurch gekennzeichnet**, daß das ethylenisch ungesättigte Monomer ein Methacrylat oder Acrylat ist.

8. Material nach einem oder mehreren der vorhergehenden Ansprüche, **dadurch gekennzeichnet**, daß es als Katalysator (c) für die Lichtpolymerisation des ethylenisch ungesättigten Monomeren ein α-Diketon, gegebenenfalls in Verbindung mit einem Amin als Reduktionsmittel enthält.

9. Material nach einem oder mehreren der vorhergehenden Ansprüche, **dadurch gekennzeichnet**, daß es als Katalysator (c) für die Kaltpolymerisation des ethylenisch ungesättigten Monomeren Amin/Peroxidsysteme enthält.

10. Material nach einem oder mehreren der vorhergehenden Ansprüche, **dadurch gekennzeichnet**, daß es als Katalysator (d) Arylsulfonsäureester, insbesondere elektronegativ substituierte Arylsulfonsäuremethylester oder substituierte Sulfoniumsalze enthält, wobei die Sulfoniumsalze der allgemeinen Formel 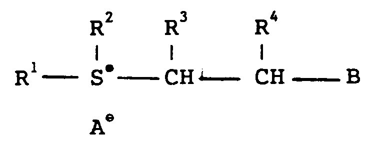 entsprechen, worin
R¹ ein Alkylrest mit 1 bis 18 C-Atomen,
R² ein Alkylrest mit 1 bis 18 C-Atomen oder Phenylalkylrest mit 7 bis 18 C-Atomen, wobei gegebenenfalls in den Alkylketten eine Estergruppe und/oder Ethergruppe enthalten sein kann, und
R³ und R⁴ jeweils ein Wasserstoffatom, ein Alkylrest mit 1 bis 18 C-Atomen und/oder ein gegebenenfalls chlor-, nitro oder alkoxysubstituierter Arylrest ist, wobei die Alkylreste R³ und R⁴ zusammen oder auch R³ oder R⁴ zusammen mit B einen cycloaliphatischen oder heterocyclischen Ring bilden können und wobei
B einen elektronenanziehenden Rest aus der Gruppe Carbonyl, Sulfonyl, Nitril, Carbonester, Chlorphenyl, Nitrophenyl, Benzoyl oder gegebenenfalls substituiertes Carbonamid und
A^{⊖} ein nicht-nukleophiles Anion darstellt.

11. Material nach einem oder mehreren der vorhergehenden Ansprüche, **dadurch gekennzeichnet**, daß es
(a) in einer Menge von 2,0 bis 35 Gew.%, vorzugsweise 2,5 bis 20 Gew.%,
(b) in einer Menge von 10 bis 80 Gew.%, vorzugsweise 15 bis 50 Gew.%,
(c) in einer Menge von 0,01 bis 5 Gew.% und
(d) in einer Menge von 0,05 bis 2,5 Gew.% enthält.

12. Material nach Anspruch 3, **dadurch gekennzeichnet**, daß es
(a) in einer Menge von 5 bis 70 Gew.%, vorzugsweise 6 bis 40 Gew.%, bezogen auf die erste Komponente,
(d) in einer Menge von 0,1 bis 5 Gew.%, bezogen auf die zweite Komponente,
enthält.

13. Verwendung eines Dentalmaterials gemäß den Ansprüchen 1 bis 12 zur Herstellung von
- Zahnersatzmaterial für temporäre Kronen und Brücken, als temporäres Füllungsmaterial sowie als Proinlay-Material für die CAM-Frästechnik und
- Composite-Zement zur Einzementierung eines zahnärztlichen Werkstücks.

## Claims

1. Dental material which is curable in a first stage to an elastic phase in which the material can be worked mechanically or surpluses can be removed, and in a second stage to its final form, containing
(a) at least one polyfunctional epimine (aziridine),
(b) at least one ethylenically unsaturated monomer,
(c) at least one catalyst for the hot, cold or light polymerization of the ethylenically unsaturated monomer and
(d) at least one catalyst to accelerate the polymerization of the epimine (a) which does not, however, influence the polymerization of (b).

2. Material according to Claim 1, **characterized in that** it is present in two separate components, namely in a first or base component and in a second or base activator component, which are mixed together on use, preferably in a ratio of 1:1.

3. Material according to Claim 2, **characterized in that**
(a) is contained only in the first component,
(b) is contained at least in the first and preferably also in the second component,
(c) is contained at least in the first component and
(d) is contained only in the second component.

4. Material according to one or more of the preceding claims, **characterized in that** it moreover contains at least one inorganic and/or organic filler.

5. Material according to one or more of the preceding claims, **characterized in that** the elastic phase remains stable for a practice-relevant period of at least approximately 3 to 30 minutes.

6. Material according to one or more of the preceding claims, **characterized in that** the polyfunctional epimine (a) is an ethylenimine compound in which on average more than one ethylenimine radical of the general formula 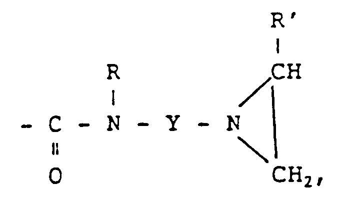 in which R and R' are a hydrogen atom or an alkyl radical and Y is a bivalent organic radical, has been introduced on the ends or in side chains of essentially linear polyethers, polythioethers or saturated polyesters with an average molecular weight of 1000 to 25,000.

7. Material according to one or more of the preceding claims, **characterized in that** the ethylenically unsaturated monomer is a methacrylate or acrylate.

8. Material according to one or more of the preceding claims, **characterized in that** it contains an α-diketone, optionally in combination with an amine as reducing agent, as catalyst (c) for the light polymerization of the ethylenically unsaturated monomer.

9. Material according to one or more of the preceding claims, **characterized in that** it contains amine/peroxide systems as catalyst (c) for the cold polymerization of the ethylenically unsaturated monomer.

10. Material according to one or more of the preceding claims, **characterized in that** it contains as catalyst (d) aryl sulphonic acid esters, in particular electronegatively substituted aryl sulphonic acid methyl esters or substituted sulphonium salts, in which the sulphonium salts correspond to the general formula 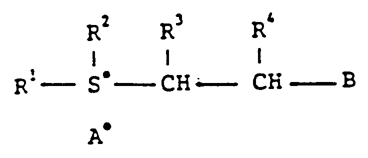 in which
R¹ is an alkyl radical having 1 to 18 C atoms,
R² is an alkyl radical having 1 to 18 C atoms or phenyl alkyl radical having 7 to 18 C atoms, in which an ester group and/or ether group can optionally be contained in the alkyl chains, and
R³ and R⁴ are in each case a hydrogen atom, an alkyl radical having 1 to 18 C atoms and/or an optionally chloro-, nitro- or alkoxy-substituted aryl radical, in which the alkyl radicals R³ and R⁴ together or also R³ or R⁴ together with B can form a cycloaliphatic or heterocyclic ring, and in which
B is an electron-attracting radical from the group carbonyl, sulphonyl, nitrile, carboxylic acid ester, chlorophenyl, nitrophenyl, benzoyl or optionally substituted carboxamide and
A^{⊖} is a non-nucleophilic anion.

11. Material according to one or more of the preceding claims, **characterized in that** it contains
(a) in a quantity of 2.0 to 35 % by weight, preferably 2.5 to 20 % by weight,
(b) in a quantity of 10 to 80 % by weight, preferably 15 to 50 % by weight,
(c) in a quantity of 0.01 to 5 % by weight, and
(d) in a quantity of 0.05 to 2.5 % by weight.

12. Material according to Claim 3, **characterized in that** it contains
(a) in a quantity of 5 to 70 % by weight, preferably 6 to 40 % by weight, relative to the first component,
(d) in a quantity of 0.1 to 5 % by weight, relative to the second component.

13. Use of a dental material according to Claims 1 to 12 for the production of
- tooth replacement material for temporary crowns and bridges, as temporary filling material and as proinlay material for the CAM milling technique, and
- composite cement for the cementing-in of a dental workpiece.

## Revendications

1. Matériau dentaire, qui est durcissable dans une première étape en une phase élastique, permettant de travailler mécaniquement le matériau ou d'éliminer les excès, et, dans une seconde étape, en sa forme finale, contenant
(a) au moins une épimine (aziridine) polyfonctionnelle,
(b) au moins un monomère à insaturation éthylénique,
(c) au moins un catalyseur pour la polymérisation à chaud, à froid ou à la lumière du monomère à insaturation éthylénique, et
(d) au moins un catalyseur qui accélère la polymérisation de l'épimine (a), mais qui n'a pas d'effet sur la polymérisation de (b).

2. Matériau selon la revendication 1, caractérisé en ce qu'il se présente sous forme de deux constituants séparés, à savoir un premier constituant ou constituant de base et un second constituant ou constituant activateur de base, que l'on mélange au moment de l'emploi, de préférence en un rapport de 1:1.

3. Matériau selon la revendication 2, caractérisé en ce que
(a) n'est contenu que dans le premier constituant,
(b) est contenu dans le premier et de préférence aussi dans le second constituant,
(c) est contenu au moins dans le premier constituant, et
(d) n'est contenu que dans le second constituant.

4. Matériau selon l'une ou plusieurs des revendications précédentes, caractérisé en ce qu'il contient en outre au moins une charge inorganique et/ou organique.

5. Matériau selon l'une ou plusieurs des revendications précédentes, caractérisé en ce que la phase élastique reste stable pendant un espace de temps significatif pour la pratique d'au moins environ 3 à 30 minutes.

6. Matériau selon l'une ou plusieurs des revendications précédentes, caractérisé en ce que l'épimine polyfonctionnelle est (a) un composé d'éthylène-imine pour lequel ont été introduits, aux extrémités ou dans des châines latérales de polyéthers, polythioéthers ou polyesters saturés essentiellement linéaires ayant une masse molaire moyenne de 1 000 à 25 000, en moyenne plus d'un reste éthylène-imine de formule générale 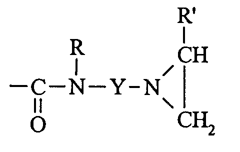 dans laquelle R et R' représentent un atome d'hydrogène ou un reste alkyle et Y représente un reste organique divalent.

7. Matériau selon l'une ou plusieurs des revendications précédentes, caractérisé en ce que le monomère à insaturation éthylénique est un méthacrylate ou un acrylate.

8. Matériau selon l'une ou plusieurs des revendications précédentes, caractérisé en ce qu'il contient une α-dicétone, éventuellement en combinaison avec une amine en tant qu'agent réducteur, comme catalyseur (c) pour la polymérisation à la lumière du monomère à insaturation éthylénique,.

9. Matériau selon l'une ou plusieurs des revendications précédentes, caractérisé en ce qu'il contient des systèmes amine/peroxyde comme catalyseur (c) pour la polymérisation à froid du monomère à insaturation éthylénique.

10. Matériau selon l'une ou plusieurs des revendications précédentes, caractérisé en ce qu'il contient comme catalyseur (d) des esters d'acides arylsulfoniques, en particulier des arylsulfonates de méthyle à substituants électronégatifs ou des sels de sulfonium substitués, les sels de sulfonium correspondant à la formule générale 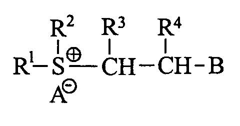 dans laquelle
R¹ représente un reste alkyle de 1 à 18 atomes de carbone,
R² représente un reste alkyle de 1 à 18 atomes de carbone ou un reste phénylalkyle de 7 à 18 atomes de carbone, les chaînes alkyle pouvant éventuellement contenir un groupe ester et/ou un groupe éther, et
R³ et R⁴ représentent chacun un atome d'hydrogène, un reste alkyle de 1 à 18 atomes de carbone et/ou un reste aryle éventuellement substitué par chloro, nitro ou alcoxy, les restes alkyle R³ et R⁴ ensemble ou encore R³ ou R⁴ avec B pouvant former un noyau cycloaliphatique ou hétérocyclique, et
B représente un reste attracteur d'électrons du groupe constitué par les restes carbonyle, sulfonyle, nitrile, ester carboxylique, chlorophényle, nitrophényle, benzoyle ou carboxamide éventuellement substitué, et
A^{⊖} représente un anion non nucléophile.

11. Matériau selon l'une ou plusieurs des revendications précédentes, caractérisé en ce qu'il contient
(a) en une quantité de 2,0 à 35 % en masse, de préférence de 2,5 à 20 % en masse,
(b) en une quantité de 10 à 80 % en masse, de préférence de 15 à 50 % enmasse,
(c) en une quantité de 0,01 à 5 % en masse, et
(d) en une quantité de 0,05 à 2,5 % en masse.

12. Matériau selon la revendication 3, caractérisé en ce qu'il contient
(a) en une quantité de 5 à 70 % en masse, de préférence de 6 à 40 % en masse, par rapport au premier constituant,
(d) en une quantité de 0,1 à 5 % en masse, par rapport au second constituant.

13. Utilisation d'un matériau dentaire selon les revendications 1 à 12 pour la préparation
- d'un matériau de prothèse dentaire pour des couronnes et des bridges temporaires, comme matériau de remplissage temporaire et comme matériau de pro-inlay pour la technique de fraisage FAO, et
- d'un ciment composite pour l'enrobage par le ciment d'une pièce prothétique.
